# EUROPEAN PATENT SPECIFICATION

(11) **EP 3 099 217 B1**
(45) Date of publication and mention of the grant of the patent: **23.05.2018**
(21) Application number: 15708570.5
(22) Date of filing: 30.01.2015
(51) Int. Cl.: A61B 3/16, A61B 3/103

(54) **NON-INVASIVE COMPRESSION AND REFRACTION CONTACT TONOMETER FOR MEASURING THE PRESSURE IN THE POSTERIOR CHAMBER AND / OR THE VITREOUS CHAMBER OF THE EYE**
NICHTINVASIVER KOMPRESSIONS- UND REFRAKTIONSKONTAKTTONOMETER ZUR MESSUNG DES DRUCKS IN DER HINTEREN AUGENKAMMER UND/ODER IM GLASKÖRPERRAUM DES AUGES
TONOMÈTRE DE RÉFRACTION PAR CONTACT ET COMPRESSION NON-INVASIF DESTINÉ À MESURER LA PRESSION DANS LA CHAMBRE POSTÉRIEURE ET/OU LA CHAMBRE DU VITRÉ DE L' OEIL

(30) Priority: 31.01.2014 PL 40703914
(43) Date of publication of application: 07.12.2016
(73) Proprietor: Piotr Nogal "Twoje Oczy", 55-100 Trzebnica (PL)
(72) Inventor: NOGAL, Piotr, 55-100 Trzebnica (PL)
(74) Representative: Witek, Rafal
(86) International application number: PCT/IB2015/000092
(87) International publication number: WO 2015/114446

(56) References cited:
- DE-A1-102004 019 804
- US-A1- 2005 182 312
- US-A1- 2007 173 713
- US-A1- 2013 271 727

## Description

The invention is a non-invasive compression and refraction contact tonometer for measuring the intraocular pressure of the anterior chamber and/or the vitreous chamber of the eye used in medical diagnostics. Intraocular pressure in the eye when increased constitutes an etiopathogenic factor for numerous ophthalmological, as well as systemic human disorders.

The interior space of the eyeball filled with aqueous humor is divided anatomically and physiologically into two chambers. The anterior chamber of the eye is a space defined anatomically and physiologically by the cornea (front), the iris and the lens in projection of the pupil from the rear. The posterior chamber of the eye is a space limited by the lens and the iris from the front, laterally by the ciliary body and by the vitreous from the back. The aqueous humor, which conditions the intraocular pressure, is produced in the posterior chamber and it goes through the pupil, between the iris and the lens, into the anterior chamber of the eye, from where it flows into the veins. The point of contact between the iris and the lens at the pupillary edge constitutes a pressure reduction valve, the tightness of which is conditioned by a pressure gradient between the anterior and posterior chamber of the eye. Pathophysiological situations associated with the blockage of the valve ("pupillary block") occur, in which the pressure gradient, which conditions the flow between the chambers, is significantly increased. The pressure in the posterior chamber and vitreous chamber grows significantly, while the pressure in the anterior chamber remains relatively low, which makes the pressure measurements in the anterior chamber inadequate to the pressure measurement in the posterior chamber and the vitreous chamber. Considering the lack of significant for the intraocular pressure anatomical and physiological limitations between the posterior chamber and the vitreous chamber, it can be assumed that the pressure of the aqueous humor in the posterior chamber always equals the pressure of the neighboring vitreous humor, which as it is more dense than the aqueous humor, under the pressure of the aqueous humor in the posterior chamber, affects the internal walls of the eyeball, in the projection of the vitreous chamber, with the same force as the aqueous humor affects it. It can be concluded that the pressure measurement in the posterior chamber and the vitreous can take place objectively in accordance with the Imbert-Fick principle as a consequence of the impression on the wall of the eyeball in any place to the back from the base of the iris in the projection of the posterior chamber and the vitreous on this wall. However practically, the impression must take place beyond the projection of the ciliary wreath; it occurs 2 mm towards the back from the corneal limbus (variable ciliary muscle tension conditions the stiffness of the wall of the eyeball at this point in a unitless way), beyond the extraocular muscles area; 5.5 mm towards the back from the corneal limbus (the impression on the bodies and attachments is caused by their contraction and makes the measurement inadequate to the stiffness of the wall of the eyeball beneath them) and theoretically beyond the optic nerve disc (no access to this point or to the nerve coming out of the eyeball), which considering the assumed measurement condition as a measurement non-invasive to the eyeball and the eye socket, determines the most convenient area of impression in the projection of the flat part of the ciliary body, which is 2-6 mm from the corneal limbus, and when the above mentioned conditions are fulfilled, 2-5 mm towards the back from the corneal limbus, which additionally allows to avoid impression in the projection of the base of the vitreous. Frontal location of the impression considering the circumference of the eyeball allows to obtain deformation of the cornea with a lot less pressure force than with the impression in the area around the circumference or to the back of it.
In the previously used tonometers the intraocular pressure value in the eyeball was estimated on the basis of measurement of this parameter in the anterior chamber. Document DE 10 2004 019804 describes such a standard non-invasive contact compression tonometer for measuring the intraocular pressure of the eye bulbus according to the preamble of claim 1. While it is obvious that considering the pupillary block, an abnormal increase of the intraocular pressure in the posterior chamber of the eye and the vitreous chamber can reach values of tens and more mmHg with the normal pressure in the anterior chamber and activate pathophysiological mechanisms, leading to serious threats to the health of the eye (e.g. closed and open angle glaucoma), as well as of the entire system (migraines). Therefore it is necessary to construct a device which allows to measure the intraocular pressure of the posterior chamber of the eye.

In the previously constructed tonometers attention was paid to the construction of the most precise measuring device which considering their anatomical limitations of the measuring point on the eyeball in the eye socket and the lack of a diagnostically effective measuring method, would evaluate the intraocular pressure in the eye on the basis of the measurement of this parameter in the anterior chamber of the eye. In impression contact tonometers (Goldmann applanation tonometer, Perkins and Pascal's tonometer, and Schiotz's tonometer) and impression non-contact tonometers (non-contact air "puff" tonometer, Ocular response analyzer) which operate in accordance with the Imbert-Fick principle, the external force is applied during the measurement to the cornea, of which parameter changes secondary to this force are measures and relativized according to the pressure in the eye behind the cornea, which considering the pressure gradient between the chambers, provides for the intraocular pressure measurement only in the anterior chamber of the eye.
Moreover, in the majority of known devices the readings of the intraocular pressure value is only possible in situations when the central part of the cornea is in good condition, and therefore impossible in pathological situations related to some disorders of the central part of the cornea (e.g. central corneal ulcer or keratoprosthesis).
In all known ophthalmological contact tonometers contact between the device and the cornea takes place during the measurement - the contact/measuring element compresses the cornea from which the measurement is read. It provides for the possibility of corneal damage and infection during the measurement.
In a different type of tonometer (Diaton tonometer) the measurement is taken, having compressed the eyeball with a free measuring stick in the projection of the ciliary wreath via the upper eyelid shield. Up until now it has been the only registered tonometer which can measure the pressure beyond the cornea. The values of the intraocular pressure are estimated on the basis of the relativization of the intraocular pressure according to the pulsation of the eyeball walls which occurs as a result of the pulsatile nature of the aqueous humor flow from the eye through the veins, conditioned by variable breathing, heart rate and blood pressure values. Stylus reflection through the upper eyelid shield conditioned by the force of pulsatile wave reflection of the eyeball wall, which depends on numerous factors - not only the intraocular pressure, is being measured. Indirectly the measurement concerns the pressure of the entire eyeball without the possibility to assess how it concerns individual chambers of the eye. Additionally in this case the measurement takes place through the skin, muscles and eyelid shield, which constitute a flexible partition, which depending on their condition can affect the precision of the measurement. In this case the precision of the measurement can be also affected by ciliary muscle tension inside the eyeball, as well as blood pressure, heart rate and respiration parameters, which provide for additional components affecting the amplitude value of the eyeball pulsation being measured. The non-contact "air-puff" tonometer in order to flatten the cornea (the measurement concerns the anterior chamber) uses an air puff directed at highly innervated cornea, which in many cases provides for measurement inaccuracy and still requires local anesthesia of the cornea considering the instinctive orbicularis muscle tension, or the fact that patients move away from the device.
While other previously used contact tonometers (Schiotz, Goldmann, Perkins, Pascal) force the person conducting the measurements to perform local anesthesia of the eye considering the contact of the device with the highly innervated cornea, which provides for problems with conducting measurement in patients allergic to the drug, or organizational limitations of the diagnostic process.
The only previously described tonometers which measure the intraocular pressure only in the posterior chamber of the eye turn out to be experimental and very expensive electronic implants for the posterior chamber of the eye with pressure receptors, which send the measured pressure values to the receivers outside the eyeball. Most of all it requires the implantation of such equipment into the eye violating the continuity of the eyeball wall, which in the case of penetration into the posterior chamber through the flat part of the ciliary body increases the risk of serious complications in every case, e.g. endophthalmitis and subsequent blindness.

The purpose of the invention is to deliver a solution which abolished previous faults in the technical condition. The purpose of the invention is to deliver a tonometer which measures in a manner non-invasive to the eyeball the intraocular pressure in the posterior chamber of the eye and the vitreous chamber, which allows the measurement to be conducted in an easier way and significantly minimalize the risk of complications related to damaging the continuity of the eyeball coatings, especially in the posterior segment, as well as to lower the costs of production of the device and the measurement itself. The purpose of the invention is to deliver a tonometer which can be used without any special diagnostic conditions in a patient with acute conditions, in whom increased intraocular pressure in the posterior chamber of the eye and the vitreous chamber is suspected, when the pressure of the anterior chamber is normal, as a reason for acute conditions, e.g. in the migraine diagnosis.

The invention is a non-invasive contact compression and refraction tonometer to measure the intraocular pressure of the posterior chamber of the eye and the vitreous chamber, characterized by a head (1) with a compression actuator (5) connected to compression forceps (8) and compression arms (11) with two symmetrical compression feet (14), crescent-shaped so that their curvature of the contact surface with the wall of the eyeball correspond to the curvature occurring in the eyeball beyond the cornea, beneficially 3-5 mm toward the back from the corneal limbus (12), compressing the eyeball beneficially at 3 and 9 o'clock direction; moreover it has a compression engine (5) connected by a two-phase connector with an optical meter (10) and a newton meter (7) with separate switches (3) and (4) of a compression actuator (5) operation mode.
Beneficially, the optic meter measuring corneal astigmatism is a keratometer or a kerato-refraktometer.
Beneficially, the head (1) is set relative to the eye using distance actuators (17) controlled by a computer (15).
Beneficially, the optic meter is equipped in a cornea distance sensor (12) which sends a signal to the computer (15) via a connector when the head is approaching the eye using distance actuators (17).
Beneficially, the signal from the optic meter (10) and the newton meter (7) is received by a registering device (6) and (9), which forwards the signal to the computer (15).
Beneficially, the actuator (5) operation mode activates the switch (2).
Beneficially, the signal from the computer (15) is sent via the connector (16) to the distance actuators (17).
Beneficially, the tonometer has an optic meter (18) with a pachymeter and pressure tonometer in the anterior chamber of the eye.
Beneficially, the data regarding the pachymetry and pressure values in the anterior chamber of the eye are entered manually.
Beneficially, the compression feet (14) are single-use or covered with single-use overlays.

The tonometer is the first compression device used in the eyeball, as the paired compression arms perform symmetrical impressions directed concentrically at both sides of the eyeball, which leads to its compression.
The tonometer is a contact device which as the first device to use the measurable corneal deformation, having compressed the eyeball wall beyond the cornea, to measure intraocular pressure in a safe distance towards the back of the corneal limbus. In none of the previously used tonometers the measurement of corneal astigmatism induced by compressing the eyeball was used.
In the device the external (impression) force applied on the eyeball in a contact manner beyond the cornea (a few millimeters towards the back of its limbus), which remains within the eyeball wall anatomically connected to the posterior chamber of the eye and the vitreous chamber, which allows to measure intraocular pressure in the chambers. Non-contact readings of the corneal optic changes (connected anatomically with the anterior chamber) take place with the compression beyond its area in a manner relativized according to its deformation, as the frontal part of the wall of the eyeball, which is conditioned by the deformation of the non-compressed cornea directly proportional to the compression of the wall anatomically and physiologically connected with another internal space of the eye (the posterior chamber and the vitreous chamber), anatomically and physiologically constituting an integral external part of the wall of the eyeball as the cornea.
Within the solution the readings of the values of the intraocular pressure in the posterior chamber and the vitreous chamber require only the optimal condition of the peripheral portion of the cornea, as the corneal cylindrical deformation, induced by the force applied and then measured, can be measured with a reflex from the corneal limbus using an optic meter which can measure the proximity of the corneal measuring points in particular axis in such situations.
The device guarantees a more precise measurement, as the paired compression arms are put against the wall of the eyeball in a direct and contact way to the precisely selected point corresponding to the projection of the posterior chamber and the vitreous chamber on the wall of the eyeball between the projection of the extraocular muscle attachments from the outside on the wall and the projection of the ciliary body with the ciliary muscle from the inside on the wall (the projection of the flat part of the ciliary body are beneficially compressed) or in a different solution the wall of the eyeball in the projection of the vitreous chamber. Therefore the measurement does not depend on the condition of other anatomical parts than the wall of the eyeball and it is targeted at the pressure of the posterior chamber and the vitreous chamber, the separation of which is not really significant diagnostically; the pressure - if increased - in these two chambers is nearly equal. Therefore, in the posterior chamber of the eye the pressure is higher than in the anterior chamber of the eye, which conditions the aqueous humor flow from the posterior chamber to the anterior chamber, also because of the fact that the external wall of the anterior chamber - the cornea is much more flexible than the stiffer external wall of the posterior chamber of the eye - the sclera, the intraocular pressure value in the anterior chamber have an insignificant effect on the pressure value in the posterior chamber measured with the device. In order to reduce a potential pressure calculation error in the posterior chamber of the eye in accordance with excessive stiffness of the wall of the anterior chamber, such as is the cornea which undergoes the measurement, the device can be equipped with a lens with a non-contact corneal tonometer and pachymeter, which measure pressure in the anterior chamber and the thickness of the cornea before it measures the pressure in the posterior chamber and the vitreous chamber, and in case of difficulties with the measurement of the thickness of the cornea and the pressure in the anterior chamber of the eye, it allows to enter pachymetry values and anterior chamber pressure values manually to the computer, having conducted pachymetry and pressure measurements in the anterior chamber with another meter independent from the device, before measuring the pressure in the posterior chamber with the device. In a different version of the device without the non-contact tonometer of the anterior chamber of the eye and pachymeter, the device allows entering data into the computer of the device manually. It allows to additionally clarify the measurement thanks to a relevant formula, calculating the corneal stiffness coefficient, conditioned by its thickness and the pressure in the anterior chamber of the eye. This formula is also uploaded into a program calculating the actual value of the intraocular pressure in the posterior chamber of the eye and the vitreous chamber of the eye using an internal computer.
The device ensures the cornea remains safe, as the contact of the impression arms effects the area of the eyeball a few millimeters from the back from the cornea, and approximation of the head to the eye is subject to control.
The device is completely non-invasive to the eyeball; it does not penetrate the wall of the eyeball like the proposed posterior chamber implants which measure its pressure. The device impressively affects the wall of the eyeball beyond the cornea which allows the risk of its potential damage and infection during the measurement to be reduced.
The device allows the use of a meter, measuring the corneal astigmatism value in relation to the area adjacent to the corneal limbus, thanks to the possibility to measure the proximity of reflex measuring points from the corneal limbus in the horizontal axis and the distance of such points in the vertical axis, which corresponds with the increase of the corneal astigmatism value of the secondary to symmetrical compression of the eyeball in the horizontal axis (conversely with the compression in the vertical axis) and allows to conduct measurements despite of pathologies of the central part of the cornea, corneal prosthesis and after surgeries of the cornea correcting vision defects or after injuries of the central part of the cornea.

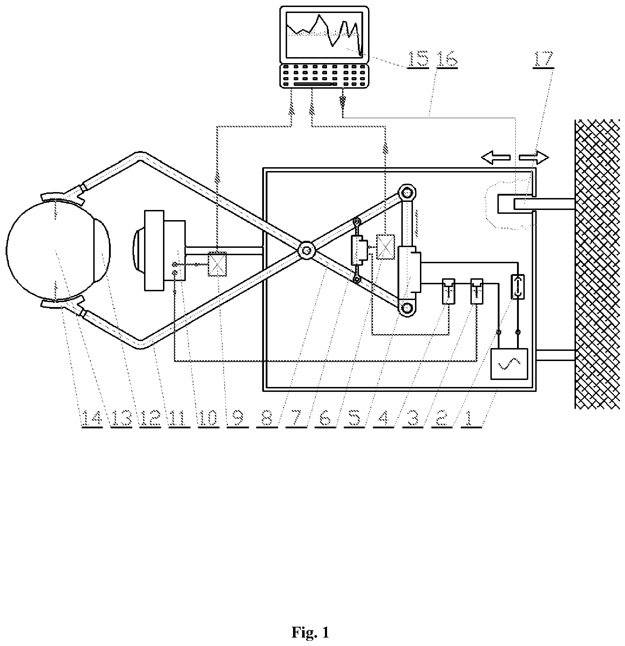

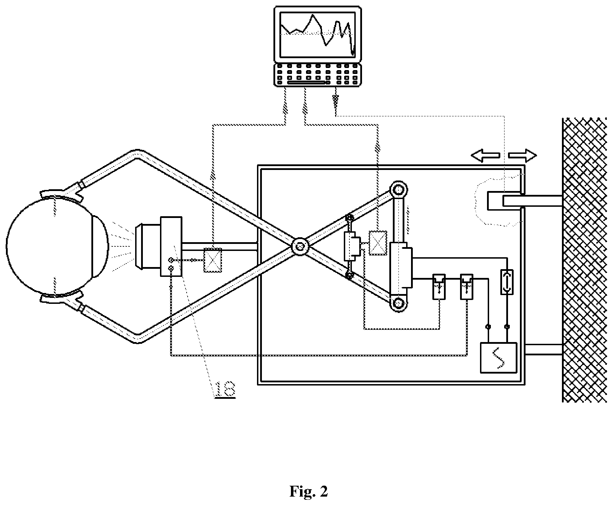

The device also allows in exceptional situations, conditioned by the lack of possibility for surface anesthesia (e.g. allergy to the anesthetic) to conduct measurements thanks to the fact that the eyeball conjunctiva, which the compression arms are put against is so poorly innervated that even without the anesthesia the measurement is possible.
The tonometer, thanks to the fact that such a compression function and a measurement reading function are divided into two coupled main elements operating in different locations in the eyeball (the compression element with the newton meter - contact in relation to the eyeball beyond the cornea and the optic meter - non-contact in relation to the eyeball within the cornea), allows to completely avoid disinfection by the use of single-use outlays installed on the compression feet, or the use of single-use compression feet, which practically eliminates the risk of infection.
An adequate measurement with the tonometer provides for the accuracy of diagnosis and effectiveness of treatment of numerous ophthalmological and systemic diseases conditioned by increased pressure in the posterior chamber of the eye and the vitreous chamber of the eye. Up until now measuring the pressure in the anterior chamber of the eye it was declared that it corresponds to the pressure in the posterior chamber of the eye and the vitreous chamber of the eye, where the increased pressure has the largest significance in causing or intensifying pathologies (e.g. compression on the optic nerve fibers in glaucoma). While it turns out that the author's development of the invention regarding the mechanism of pupillary block intensification (from relative to absolute block) and increase of the pressure in the posterior chamber and the vitreous chamber of the eye with the normal or decreased pressure in the anterior chamber of the eye sheds new light on the problem of unconditional necessity to measure pressure in the posterior chamber and the vitreous chamber of the eye as the only diagnostically effective measurement for diagnosis of numerous ophthalmological conditions (e.g. glaucoma), as well as other conditions (e.g. migraines).
The invention is presented in the example shown in a drawing in which Fig. 1 presents a non-invasive contact compression and refraction tonometer for the intraocular pressure of the posterior chamber of the eye, Fig. 2 presents a variant of the above mentioned tonometer with an optic meter with pachymeter and impression tonometer (non-contact) for the pressure in the anterior chamber of the eye.

### Example 1

The main element of the device is the head (1) axially movable in relations to the body thanks to distance actuators (17), which set the head axially closer to or further from the eye, and controlled by a signal from the tonometer's computer (15). The head (1) is equipped with the following main parts: an impression actuator (5) operation mode switch (2), an automatic switch (3) of the impression actuator operation, controlled with a signal from the optic meter (10), an automatic switch (4) of the compression actuator (5) operation mode, controlled with a signal from the newton meter (7), a compression actuator (5), a device (6) registering the signal from the newton meter (7), sending measuring data to the tonometer's computer (15), the newton meter (7) measuring the obtained compression force of the compression actuator (5), compression forceps (8) connected to the compression arms (11), a device (9) registering the signal from the optic meter (10) sending measuring data to the tonometer's computer (15), the tonometer's optic meter (10), the compression arms (11) and the compression feet (14). The tonometer's computer (15) receives a signal from the registering device (6) and (9), and sends the signal by a connector (16) to the distance actuators (17). The device allows to enter data, their recalculations and to export data to other receivers.

The tonometer operates through impression in accordance with the Imbert-Fick principle in relation to the posterior chamber of the eye (13) and the vitreous chamber of the eye, compressing the wall of the eyeball in the projection of the posterior chamber of the eye (13) and the vitreous chamber of the eye with double symmetrical compression feet (14) operating concentrically and cylindrically deforming the cornea (12), as the optically significant wall of the eyeball related to the anterior chamber, but constituting an integral part of the wall of the deformed eyeball, therefore deformed less than the impressed point, but relatively to the impression. It leads to a change of the cornea refraction (12) subject to the measurement by the optic meter of the tonometer (10). The activated compression actuator (5) connected with compression forceps (8) and compression arms (11) with two symmetrical compression feet (14) releases the centripetal compression force of the eyeball on both sides behind the corneal limbus (13) 3-5 mm at the 3 and 9 o'clock directions. It causes the compression of the eyeball and its deformation, which induces corneal astigmatism measured by the optic meter of the device (10) (Fig. 1) or in a different variant of the tonometer, by an optic meter (18) with pachymeter and non-contact impression tonometer measuring the pressure in the anterior chamber of the eye (Fig. 2).

The compression element ("Compression forceps") (8) coupled with the compression actuator (5) driving it connected by connectors with switches (3, 4) of the actuator (5) operation mode in situations when:
- the connector switch (3) with the optic meter (10) after measuring the assumed and achieved value of corneal astigmatism, secondary in relations to the eyeball compression, turns off the compression actuator (5) and further compression on the eyeball,
- the connector switch (4) with the newton meter (7) connected to the compression actuator (5) turns off the compression actuator (5) and further compression on the eyeball in the case of measuring the compression force appropriate for the intraocular pressure exceeding the assumed upper limit value of the intraocular pressure (80-100 mmHg) in relation to which obtaining the assumed cylindrical deformation of the cornea is no longer safe for the condition of the eyeball coating, while the pressure value is sufficient for the diagnostics.

The head (1) of the device can be equipped with an optic meter (18) of the tonometer with a pachymeter and non-contact impression tonometer of the pressure in the anterior chamber of the eye, which prior to measuring the pressure in the posterior chamber (13) and the vitreous chamber of the eye by the device, measure the thickness of the cornea (12) and the intraocular pressure in the anterior chamber of the eye. In a different version of the invention, the device is not equipped with an additional tonometer with pachymeter. In both versions it is possible to manually enter the value of the thickness of the cornea (12) and the pressure of the anterior chamber, measured with a separate pachymeter and tonometer of the anterior chamber of the eye undergoing measurements with the device. By design it is possible to measure two astigmatism values (e.g. 0.5D and 2.0D) or two force values (e.g. 50 uN and 100 uN), which allows to calculate the stiffness coefficient of the wall of the eyeball. It is possible to enter the pachymetry result of a particular eye into the computer. These actions allow to additionally clarify the pressure calculations in the posterior chamber of the eye (13) thanks to a formula, calculating the resistance coefficient of the cornea (12) to deformation, conditioned by the pressure in the anterior chamber of the eye, as well as by the stiffness of the walls of the eyeball.

The calculation of the intraocular pressure in the posterior chamber (13) and the vitreous chamber of the eye, or the entire eyeball (in case of good communication between chambers, e.g. after iridectomy, laser iridectomy) takes place in the tonometer's computer (15) based on two measuring methods: by a newton meter (7) measuring the force released by the impression actuator (5) necessary to obtain the initially assumed value of corneal astigmatism measure by the tonometer's optic meter (10) or the tonometer's optic meter (10) measuring the value of corneal astigmatism obtained by the impression of the eyeball using the impression actuator (5) with the initially assumed force controlled by the newton meter (7). The final calculation takes place based on the Imbert-Fick principle, which says that with the assumed impression surface on the wall of the eyeball and the known force of the impression, we can measure the pressure in the eyeball. In the device the impression surface is constituted by the sum of surfaces of the two compression feet (14), and the impression force is measured by the newton meter (7), measuring the force released by the compression actuator (5). Considering the anatomical limitations of the eyeball in the human eye socket, it is not possible to compress the eyeball on both sides of its circumference and therefore the measurement is relativized in relation to the corneal astigmatism, secondary to bilateral symmetrical impression of the wall of the eyeball ("compression") in its anterior hemisphere, measured by the optic meter.

### Example 2

Having adjusted the stands stabilizing the device in front of the eye to the frontal and zygomatic area (bones above the eye and under the eye), the person conducting the measurements blocks the stabilizing stands in order to eliminate the possibility of the body of the device to move in relation to the eye during the measurements, as it could result in the falsification of the result or in damage of the eye caused by the movable measuring elements considering their additional and uncontrolled movement in relation to the eyeball.

Activation of the device in the preparation mode results in the measuring head (1) being pulled out along with the compression arms (11) and the lens of the optic meter (10) with the distance sensor to the assumed distance from the top of the cornea (12). It also results in setting the open compression arms (11) at such a distance and under such an angle in relation to the eyeball, that after the activation of the measuring mode they will be put against the wall of the eyeball in the projection of the posterior chamber of the eye (13) and/or in the projection of the vitreous chamber of the eye in the estimated and assumed distance to the back from the corneal limbus (12).

Switching the device to the measuring mode results in resetting the measured potential initial corneal astigmatism (12), diagnosed by the optic meter before the measuring, and then in the centripetal movement of the compression arms (11) and the contact of compression feet (14) with the eyeball at the 3 and 9 o'clock directions 3-5 mm to the back from the corneal limbus (12). The compression feet (14) can be single-use or covered with single-use outlays which allows to conduct measurements without disinfecting the contact elements in relation to the eye in the device (the stabilizing stands require potential disinfection or single-use outlays, as they remain in contact with the skin around the frontal and zygomatic area (bones above the eye and under the eye).

The compression arms (11) are drawn closer thanks to the operation of the compression actuator (5) connected to them, which results in recession of the eyeball and its compression in the horizontal axis, inducing corneal astigmatism (12) measured by the optic meter (10) of the device.

The cable powering the impression actuator (5) from the power source is connected with two connectors (with switches (3), (4); one for each connector), of which the first one (3) is connected with the optic meter (1), and the second one (4) with the newton meter (7) measuring the force released by the compression actuator (5). Achieving the assumed level of the corneal astigmatism, measured by the optic meter (1), results in switching off the compression actuator (5) operation mode by the first switch (3). If for any reasons (e.g. too high intraocular pressure which makes it impossible to obtain the assumed astigmatism, or the lack of astigmatism readings by the optic meter) the operation of the compression actuator (5) was not switched off, which would result in further compression of the eyeball, the second switch (4), connected with the newton meter (7), determining the force by the newton meter (7), switches off the compression actuator (5) operation mode and stops further compression of the arms (11) with the compression feet (14) on the eyeball.

The compression arms (11) compress the eyeball using the centripetal force to achieve the initial astigmatism (e.g. 0.5D) and the target astigmatism (e.g. 2.0D), which allows to increase the measurement precision by recalculating the stiffness coefficient of the walls of the eyeball through referring the result to the table developed on the basis of measurements conducted in control groups; values of the stiffness coefficient of the walls of the eyeball calculated in such a way are entered into the program of the internal computer (15), calculating the pressure in the posterior chamber of the eye (13) and the vitreous chamber of the eye, which just after the measurement allows the result to be displayed on a LCD panel of the device.

## Claims

1. A non-invasive contact compression and refraction tonometer for measuring the intraocular pressure of the posterior chamber of the eye and/or the vitreous chamber of the eye, **characterized by** the fact that it is equipped with a head (1) with a compression actuator (5) connected by compression forceps (8) and compression arms (11) with two symmetrical compression feet (14) crescent-shaped in such a way that their curvature and contact surface with the wall of the eyeball correspond to the curvature in the eyeball beyond the corneal area, beneficially 3-5 mm to the back from the corneal limbus (12), compressing the eyeball, beneficially at the 3 and 9 o'clock directions; moreover it is equipped with a compression actuator (5) connected by a two-phase connector with an optic meter (10) and a newton meter (7) with switches (3) and (4) of the compression actuator (5) operation mode separate for each of them.

2. The tonometer according to Claim 1 is **characterized by** the fact that its optic meter, measuring corneal astigmatism, is a keratometer or a keratorefractometer.

3. The tonometer according to Claim 1 **characterized in that** the head (1) is set in relation to the eye using distance actuators (17) controlled by the computer (15).

4. The tonometer according to Claim 1 **characterized in that** the optic meter is equipped with a distance sensor from the cornea (12), which sends a signal to the computer (15) through a connector when the head is approaching the eye with the use of distance actuators (17).

5. The tonometer according to Claim 1 **characterized in that** the signal from the optic meter (10) and the newton meter (7) is received by the registering device (6) and (9), which forwards the signal to the computer (15).

6. The tonometer according to Claim 1 **characterized in that** the switch (2) activates the actuator (5) operation mode.

7. The tonometer according to Claim 1 **characterized in that** the signal from the computer (15) to the distance actuators (17) is sent by the connector (16).

8. The tonometer according to Claim 1 **characterized in that** it is equipped with the optic meter (18) with a pachymeter and a tonometer for measuring pressure in the anterior chamber of the eye.

9. The tonometer according to Claim 1 **characterized in that** data regarding the values of the pressure in the anterior chamber of the eye and pachymetry are entered manually.

10. The tonometer according to Claim 1 **characterized in that** the compression feet (14) are single-use or covered with single-use outlays.

## Patentansprüche

1. Ein nichtinvasives Kontaktkompressions- und -refraktionstonometer zur Messung des Augeninnendrucks der hinteren Augenkammer und/oder des Glaskörpers des Auges, dadurch charakterisiert, dass es mit einem Kopf (1) mit einem Kompressionsaktor (5) ausgestattet ist, der durch eine Kompressionsklemme (8) und Kompressionsarme (11) mit zwei symmetrischen Kompressionsfüßen (14) verbunden ist, die sichelförmig sind, so dass ihre Krümmung und ihre Kontaktoberfläche mit der Augapfelwand der Krümmung im Augapfel hinter der Hornhautzone entspricht, vorteilhaft 3-5 mm hinter dem Limbus (12), den Augapfel komprimierend, vorteilhaft in den 3- und 9-Uhr-Richtungen; darüber hinaus ist es mit einem Kompressionsaktor (5) ausgestattet, der durch einen Zweiphasenkonnektor mit einem optischen Messgerät (10) und einem Newtonmessgerät (7) mit für beide separaten Schaltern (3) und (4) für den Betriebsmodus des Kompressionsaktors (5) verbunden ist.

2. Das Tonometer nach Anspruch 1, dadurch charakterisiert, dass sein optisches Messgerät, das die Hornhautverkrümmung misst, ein Keratometer oder ein Keratorefraktometer ist.

3. Das Tonometer nach Anspruch 1, dadurch charakterisiert, dass der Kopf (1) im Verhältnis zum Auge eingestellt ist, mittels durch den Computer (15) kontrollierten Abstandsaktoren (17).

4. Das Tonometer nach Anspruch 1, dadurch charakterisiert, dass das optische Messgerät mit einem Sensor für den Abstand von der Hornhaut (12) ausgestattet ist, der ein Signal zum Computer (15) über einen Konnektor schickt, wenn der Kopf sich dem Auge mittels der Abstandsaktoren (17) annähert.

5. Das Tonometer nach Anspruch 1, dadurch charakterisiert, dass das Signal vom optischen Messgerät (10) und dem Newtonmessgerät (7) vom Registriergerät (6) und (9) aufgefangen wird, welches das Signal an den Computer (15) weiterleitet.

6. Das Tonometer nach Anspruch 1, dadurch charakterisiert, dass der Schalter (2) den Aktor (5)-Betriebsmodus aktiviert.

7. Das Tonometer nach Anspruch 1, dadurch charakterisiert, dass das Signal vom Computer (15) an die Abstandsaktoren (17) durch den Konnektor (16) gesendet wird.

8. Das Tonometer nach Anspruch 1, dadurch charakterisiert, dass es mit einem optischen Messgerät (18) mit einem Pachymeter und einem Tonometer für die Messung des Drucks in der vorderen Augenkammer ausgestattet ist.

9. Das Tonometer nach Anspruch 1, dadurch charakterisiert, dass Daten über die Werte des Drucks in der vorderen Augenkammer und der Pachymetrie von Hand eingegeben werden.

10. Das Tonometer nach Anspruch 1, dadurch charakterisiert, dass die Kompressionsfüße (14) zur einmaligen Verwendung sind oder mit Aufsätzen zur einmaligen Verwendung bedeckt sind.

## Revendications

1. Tonomètre à compression et réfraction à contact non invasif pour mesurer la pression intra-oculaire de la chambre postérieure de l'oeil et/ou de la cavité vitréenne de l'oeil, **caractérisé en ce qu'**il est équipé d'une tête (1) avec un actionneur de compression (5) relié par un forceps de compression (8) et des bras de compression (11) à deux pieds de compression symétriques (14) en forme de croissant de telle sorte que leur courbure et leur surface de contact avec la paroi du globe oculaire correspondent à la courbure dans le globe oculaire au-delà de la zone cornéenne, de façon bénéfique à 3 à 5 mm à l'arrière du limbe de la cornée (12), comprimant le globe oculaire, de façon bénéfique dans les directions à 3 heures et à 9 heures des aiguilles d'une montre ; de plus, il est équipé d'un actionneur de compression (5) relié par un connecteur à deux phases à un dispositif de mesure optique (10) et à un dynamomètre (7) par des commutateurs (3) et (4) du mode de fonctionnement de l'actionneur de compression (5), séparés pour chacun d'entre eux.

2. Tonomètre selon la revendication 1, **caractérisé en ce que** son dispositif de mesure optique, mesurant l'astigmatisme cornéen, est un kératomètre ou un kérato-réfractomètre.

3. Tonomètre selon la revendication 1, **caractérisé en ce que** la tête (1) est positionnée en relation avec l'oeil à l'aide d'actionneurs de distance (17) commandés par l'ordinateur (15).

4. Tonomètre selon la revendication 1, **caractérisé en ce que** le dispositif de mesure optique est équipé d'un capteur de distance par rapport à la cornée (12), qui envoie un signal à l'ordinateur (15) par l'intermédiaire d'un connecteur lorsque la tête est rapprochée de l'oeil à l'aide d'actionneurs de distance (17).

5. Tonomètre selon la revendication 1, **caractérisé en ce que** le signal venant du dispositif de mesure optique (10) et du dynamomètre (7) est reçu par le dispositif d'alignement (6) et (9), qui transmet le signal à l'ordinateur (15).

6. Tonomètre selon la revendication 1, **caractérisé en ce que** le commutateur (2) active le mode de fonctionnement de l'actionneur (5).

7. Tonomètre selon la revendication 1, **caractérisé en ce que** le signal de l'ordinateur (15) aux actionneurs de distance (17) est envoyé par le connecteur (16).

8. Tonomètre selon la revendication 1, **caractérisé en ce qu'**il est équipé du dispositif de mesure optique (18) avec un pachymètre et un tonomètre pour mesurer une pression dans la chambre antérieure de l'oeil.

9. Tonomètre selon la revendication 1, **caractérisé en ce que** des données concernant les valeurs de la pression dans la chambre antérieure de l'oeil et la pachymétrie sont entrées manuellement.

10. Tonomètre selon la revendication 1, **caractérisé en ce que** les pieds de compression (14) sont à usage unique ou recouverts de pièces perdues à usage unique.
